# EUROPÄISCHE PATENTSCHRIFT

(11) **EP 3 130 286 B1**
(45) Veröffentlichungstag und Bekanntmachung des Hinweises auf die Patenterteilung: **05.06.2024**
(21) Anmeldenummer: 16181975.0
(22) Anmeldetag: 29.07.2016
(51) Int. Cl.: A61B 5/107, A61B 1/24, A61C 9/00, G01B 11/24

(54) **AUFNAHMEVORRICHTUNG**
RECORDING DEVICE
DISPOSITIF D'ENREGISTREMENT

(30) Priorität: 11.08.2015 EP 15180555
(43) Veröffentlichungstag der Anmeldung: 15.02.2017
(73) Patentinhaber: Ivoclar Vivadent AG, 9494 Schaan (LI)
(72) Erfinder: Senti, Theresa, 9497 Triesenberg (LI); Rohner, Gottfried, 9450 Altstätten (CH); Watzke, Ronny, 6800 Feldkirch (AT); Ferilli, Antonio, 9545 Waengi (CH)
(74) Vertreter: Baldus, Oliver

(56) Entgegenhaltungen:
- WO-A1-02/00115
- JP-A- 2000 126 122
- US-A- 5 785 051
- US-A1- 2006 241 345
- US-A1- 2010 222 706
- US-A1- 2014 330 133

## Beschreibung

Die Erfindung betrifft eine Aufnahmevorrichtung, gemäß dem Oberbegriff von Anspruch 1, sowie ein Verfahren für den Betrieb einer Aufnahmevorrichtung, gemäß dem Oberbegriff von Anspruch 15.

Es ist an sich bekannt, um Aufnahmenspulen für die Auswertung von Messignalen Ballons anzuordnen, die sich aufblasen lassen. Das Aufblasen erfolgt über den Ballonanschluss, durch den hindurch auch die Anschlüsse für den Messaufnehmer geführt sind.

Eine derartige Lösung lässt sich für den medizinischen Bereich beispielsweise der DE 42 33 809 A1, aber auch beispielsweise der WO 2014/145058A1 entnehmen. Derartige Lösungen erlauben den Schutz von empfindlichen Messaufnehmern und werden beispielsweise eingesetzt, um Gehörgänge zu analysieren. Eine weitere Aufnahmevorrichtung entsprechend des Standes der Technik ist zum Beispiel auch bekannt aus US2014/0330133A1.

In manchen Fällen ist es erwünscht, die innere Struktur eines teilweise auch zerklüfteten Hohlraums zu erfassen. Hierzu wird vorgeschlagen worden, ein stereometrisches Verfahren zu verwenden, mit Scanköpfen, die voneinander um ein vorgegebenes Maß beabstandet sind. Über eine Beleuchtungsvorrichtung am Scankopf wird die zu erfassende Oberfläche des Hohlraums, der sogenannte Scanbereich, beleuchtet und der Scanbereich des Hohlraums soll so erfasst werden.

Häufig ist jedoch trotz aufwändiger Scanner das Scanergebnis unbefriedigend, so dass man versucht hat, entsprechend kostenaufwändig den Scankopf noch weiter zu verbessern. Dennoch lieferten die bislang insofern durchgeführten Versuche insbesondere bei dreidimensional kompliziert geformten Hohlräumen wenig befriedigende Ergebnisse.

Um dennoch eine einigermaßen präzise Erfassung der Oberfläche des Hohlraums, also des Scanbereichs zu ermöglichen, ist es bereits vorgeschlagen worden, mit unterschiedlichen Frequenzbereichen der elektromagnetischen Strahlung zu arbeiten. Bei einer feuchten Oberfläche kann es beispielsweise günstig sein, sichtbares Licht oder UV-Licht zu verwenden. Hingegen sind die Reflexionseigenschaften bestimmter Materialien, aus denen der Hohlraum bestehen kann, einerseits bei Ultraschall-, andererseits aber auch bei Röntgenstrahlung besser.

Nachteilig hierbei ist es, dass Scanner mit unterschiedlichen Frequenzbereichen bereitgestellt werden müssen, was die Lösung insgesamt verteuert und teilweise auch nicht mehr praktikabel macht.

Diese Lösungen sind grundsätzlich für die Erfassung der statischen Mundsituation geeignet. Es mangelt jedoch an einer Lösung für die Funktionsabformung, also die Abformung der Mundsituation unter Berücksichtigung der Verformung von Mundpartien in unterschiedlichen Stellungen des Unterkiefers zum Oberkiefers.

Man hat versucht, kurzerhand eine Abformung bei unterschiedlichen Positionen oder zwei unterschiedlichen Öffnungsstellungen des Mundes je durch zuführen. Insgesamt ist das Bewegungsmuster aufgrund der je anatomisch vorgegebenen unterschiedlichen Ausstattungen der Kondylengelenke ausgesprochen komplex und insofern nicht ohne weiteres der Digitalisierung zugänglich.

Demgegenüber liegt der Erfindung die Aufgabe zugrunde, eine Aufnahmevorrichtung gemäß dem Oberbegriff von Anspruch 1 sowie ein Verfahren zur Inbetriebnahme einer Aufnahmevorrichtung gemäß dem Oberbegriff von Anspruch 15 zu schaffen, die auch für die Funktionsabformung gut, und jedenfalls deutlich besser als die Aufnahmevorrichtungen des jetzigen Stands der Technik, geeignet sind.

Diese Aufgabe wird erfindungsgemäß durch die Ansprüche 1 bzw. 15 gelöst.

Vorteilhafte Weiterbildungen ergeben sich aus den Unteransprüchen.

Es ist vorgesehen, dass das folienartige Material nach der Art eines Ballons ausgebreitet ist und sich insbesondere über einen Kieferkamm im Mund eines Patienten erstreckt, aber auch in der Mundhöhle im übrigen. In diesem Ballon ist ein erster Scankopf eingebracht. Wenn der Ballon unter Überdruck gesetzt wird, liegt er an dem Scanbereich an, also beispielsweise am unbezahnten Kieferkamm des Patienten. Durch den Überdruck wird Weichgewebe verformt, während Hartgewebe wie bestehende Zähne nicht oder nicht wirklich verformt wird.

Unter Scankopf seien hier Erfassungsvorrichtungen beliebiger Art zu verstehen, über welche mittelbar oder unmittelbar ein 3D-Modell des gescanten Objekts erzeugt werden kann, z, B. ein Scankopf unter Verwendunge des Dopplereffekts, auch OCT genannt, einer unter Verwenung elektomagnetischen Strahlung, z.B. sichtbares oder unsichtbares Licht oder Röntgenstrahlung, insbesondere ein Array aus CCD-Chips, oder auch Ultraschall.

Das Ausmaß der Verformung hängt von dem aufgebrachten Überdruck ab. Wenn der Überdruck erhöht wird, erfolgt eine stärkere Verfomung. Es ist beabsichtigt, die Verformung bei entsprechender Wahl des Überdrucks vorzunehmen, so dass die Aufnahmevorrichtung, die eine Scanvorrichtung umfasst, erkennen kann, ob an den betreffenden betrachteten Scanbereichen Weichgewebe oder Hartgewebe vorliegt.

Hierzu ist bevorzugt ein Muster auf dem folienartigen Material aufgebracht. Das Muster lässt sich vom ersten Scankopf erkennen und die Verformung während der Druckbeaufschlagung des folienartigen Materials lässt sich erfassen.

Es ist vorgesehen, dass ein zweiter Scankopf von dem ersten Scankopf beabstandet ist. Alternativ ist es auch möglich, einen Scankopf mit mehreren Scaneinheiten zu realisieren, von denen eine intraoral und eine andere extraoral angeordnet ist, so dass gleichzeitig die anatomischen Gegebenheiten in dem Mund und außerhalb des Mundes erfasst werden können. Während der erste Scankopf wie ausgeführt bevorzugt in dem Ballon im Mund des Patienten angeordnet ist, ist der zweite Scankopf außerhalb des Mundes des Patienten angeordnet und richtet sich auf diesen. Die Ausrichtung erfolgt so, dass die Scanachse im wesentlichen zum ersten Scankopf hingerichtet ist.

Es ist auch möglich, für den intraoralen und den extraoralen Scankopf unterschiedliche Scanverfahren zu verwenden. Lediglich beispielhaft könnte als intraoraler oder erster Scankopf ein Scankopf mit optischer Kohärenztomographie (OCT) verwendet werden, und extraoral ein stereoskopischer Scankopf.

Der Erfassungsbereich erstreckt sich um diese Scanachse herum, beispielsweise, so dass zumindest der Bereich der Lippen des Patienten vollständig erfassbar ist.

Es ist auch möglich, den Scanbereich wesentlich über diesen Bereich hinaus auszudehnen, z.B. wenn der Tragus oder ein Markierungselement am Tragus und/oder die Bipupillarlinie erfasst werden soll. Wenn der zweite Scankopf beispielsweise in einem Abstand von 10cm angeordnet ist reicht ein Scanwinkel von +/- 45 Grad in jede Richtung ohne weiteres aus, um den Mund des Patienten vollständig abzudecken.

Durch den zweiten Scankopf wird erfasst, wie sich das den Mund des Patienten umgebende Gewebe, insbesondere also auch die Lippen, verformen, während der Ballon aufgeblasen wird.

Die so gewonnen Daten, also Intraoral-Daten basierend auf dem ersten Scankopf und Extraoral-Daten basierend auf dem zweiten Scankopf, werden nur elektronisch zusammengefasst und aus der Gesamtschau ergibt sich dann, wie das Weichgewebe des Patienten im Mundbereich verformbar ist.

Die Erfassung erfolgt bevorzugt so, das der Ballon stufenweise aufgeblasen wird und dann die entsprechenden 3D-Daten je erfasst werden und beispielsweise bei 1/3 Nenndruck, 2/3 Nenndruck bzw. dem vollen Nenndruck.

In vorteilhafter Ausgestaltung ist es vorgesehen, dass anschließend hieran der Druck im Ballon leicht reduziert wird, bspw. auf 80% des Nenndrucks. Der Patient soll dann Funktionsbewegungen ausführen, wodurch er den Ballon komprimiert. Diese Bewegungen werden von beiden Scanköpfen aufgezeichnet beziehungsweise die hieraus sich ergebenden Verformungen des Weichgewebes werden aufgezeichnet.

Der intraorale oder erste Scankopf erfasst nun die Punkte der Bewegung des Musters und des folien-artigen Gewebes die entsprechenden Funktionsbewegungen entsprechen den Bewegungen, die der Patient macht. Gegebenenfalls ist es hierbei erforderlich, den Druck im Ballon zu regeln, da die durch die Bewegung injizierte Luftverdrängung ausgeglichen werden sollte.

Der Extraoral-Scanner erfasst erfindungsgemäß bevorzugt die Verformung des Weichgewebes, das den Mund extraoral umgibt. Zusätzlich kann auch beispielsweise die Oklusionsebene erfasst werden, indem die Pupillen und eine Markierung, die am Tragus angebracht wird, zusätzlich erfasst wird. Bei dieser bevorzugten Ausgestaltung der Erfassungsbereiche des Extraoral-Scankopfes in entsprechender Weise gewählt, so dass auch der Augen/Ohrbereich des Patienten abgedeckt werden kann.

Basierend auf den Scanergebnissen beider Scanköpfe während der Funktionsbewegungen wird dann ein digitales Funktionsmodell erstellt, das nicht nur die Kaubewegungen erfasst, sondern auch den relevanten Bereich des Patientenkopfes.

Die Oklusionsebene lässt sich basierend auf der Distanz zwischen der Mitte der Lippen und den Tragi entsprechend dem Scannen des Extrakorporalen-Scankopfes als Anhaltspunkt für die Zahnaufstellung verwenden. Die Verformung von Lippen und Wangen erlauben es, eine Anpassung an die patientenspezifische Anatomie bei der Herstellung einer Prothese vorzunehmen. Auch die erfassten Kinnbewegungen lassen sich für die Optimierung der Zahnaufstellung verwenden.

Während der Extraoral-Scankopf für die Erfassung und Visualisierung der Lippenfülle, aber auch von Ästhetik-Linien, wie der Mittellinie, der Eckzahnlinie, der Lachlinie und der Lippenschlusslinie, erfindungsgemäß verwendet werden kann, wird durch den Intraoral- Scanner der Funktionsrand und die Oberfläche der Zahnlosen Kiefer erfasst.

Die Kieferrelation lässt sich durch gleichzeitiges Scannen von Ober- und Unterkiefer bestimmen. Es ist günstig, wenn während des Scanvorgans beider Scanköpfe mit variablem Druck gearbeitet werden kann.

Wenn der Innendruck im Ballon reduziert wird, neigt der Patient von sich aus dazu, den Mundöffnungswinkel zu reduzieren. Entsprechende Bewegungen der Kiefer des Patienten relativ zueinander führen zu einer Veränderung des Eindruck-Musters bzw. der Eindrucktiefe des folienartigen Materials, so dass auch die Verformung des zugehörigen Weichgewebes, an dem das folienartige Material unter Bereitstellung des Scanbereichs anliegt, erfasst werden kann.

Die Verwendung der erfindungsgemäßen Scanvorrichtung oder Aufnahmevorrichtung ist nicht auf Patienten mit zahnlosen Kiefern beschränkt. Auch bei teilbezahnten Kiefern lässt sich eine Bewegung des Weichgewebes im Vergleich zur Nicht-Bewegung des Hartgewebes ohne weiteres erfassen. Besonders günstig ist es wenn das folien-artige Material besonders flexibel ist und so markiert ist, dass seine Bewegungen durch den Scankopf auf Anhieb erfasst werden können.

Hierzu kann ein entsprechendes Muster auf dem folien-artigen Material aufgebracht sein. Es kann auch ein Netz oder ein anderes flächiges Material anstelle dessen verwendet werden, das dafür geeignet ist, von mindestens einem Scankopf erfasst zu werden.

Es erlaubt die Realisierung von zwei Scanköpfen auch eine dreidimensionale Aufnahme mindestens in dem Bereich, der sich im wesentlichen zwischen den Scanköpfen erstreckt, also dem Bereich der Front-/Eckzähne sowie der zugehörigen Schleimhäute und der Lippen.

Während die Erfassung der Verformbarkeit bevorzugt unter Verwendung eines einzigen Ballons realisiert wird, ist es in einer alternativen Ausgestaltung auch möglich, anstelle dessen den Ballon in einen Ballon mit mehreren Kammern aufzuteilen. Diese können dann unabhängig voneinander befüllt werden, so dass unterschiedliche Oralbereiche des Patienten je mit Luft befüllbar sind.

Es ist vorgesehen, den ersten Scankopf auf einem Scankopf-Träger zu lagern. Der zweite Scankopf kann ebenfalls auf dem gleichen Scankopf-Träger gelagert sein, oder aber auf einem separaten Scankopf-Träger. Der erste Scankopf ist für einen Rundum-Scan bestimmt, also für die Erfassung eines Bildes, das sich kugelförmig um den Scankopf erstreckt. Mindestens teilweise erstreckt sich insofern ein Scanbereich um den Scankopf herum.

Ein folienartiges Material nach der Art eines Ballons liegt an dem Scanbereich an.

Es wird unter Überdruck gesetzt was möglich ist, da der Ballon einen geschlossenen und verformbaren Körper bildet.

Die Art und Weise, auf welche der Überdruck erzeugbar ist, lässt sich in beliebiger Weise an die Erfordernisse anpassen. Bevorzugt insofern ist eine kleine Handpumpe, über welcher der Bediener gezielt einen gewissen Überdruck erzeugen kann. Alternativ ist auch die Realisierung einer maschinellen Pumpe, insbesondere einer automatisch druckgeregelten Pumpe möglich. Diese kann mit einem Manometer und/oder einem Ablassventil verbunden sein, so dass auch gezielt vorgegebene Überdruckwerte eingestellt werden können.

Der Ballon und der erste Scankopf sind bei dieser Ausführungsform intraoral angeordnet. Es versteht sich, dass eine entsprechende Realisierung auch bei einem Kopfmodell eines Patienten, das das Gewebe des Patienten nachbildet, realisiert werden kann.

Durch den Überdruck verformt das folienartige Material ein Weichgewebe, das von dem Ballon unter Druck gesetzt wird.

Von der Mundöffnung des Patienten beanstandet, jedoch auf diese ausgerichtet, ist ein weiterer Scankopf vorgesehen. Dieser Scankopf erfasst Bewegungen der Lippen, des Mundes und auch des gesamten unteren Gesichts des Patienten. Für die Vereinfachung der Erfassung können in beliebiger Weise Markierungen in und am Mund des Patienten vorgesehen sein.

Es versteht sich, dass auch beliebige andere Markierungen, beispielsweise im Bereich der Tragi, und in anderen beliebigen Stellen, möglich sind.

Es ist auch möglich, den Ballon oder eine separate Kammer des Ballons so auszugestalten, dass sie den Bereich des Mundvorhofs erfassen und ausfüllen. Dies bedingt eine entsprechende Formausgestaltung des Ballons, der insofern der Form des Alveolarfortsatzes folgt. Gegebenenfalls kann auch hier ein dritter Scankopf ausgebildet sein oder einer der beiden anderen Scanköpfe ist an diese Stelle bewegbar.

Das folienartige Material ist bevorzugt als Einwegartikel realisiert und auch preisgünstig herzustellen.

Es kann im Grunde ein Standard-Ballon mit einem entsprechend angebrachtem Aufdruck zur Bereitstellung eines Musters verwendet werden. Ein derartiger Ballon ist, wenn er oral aufgenommen ist, in geeigneter Weise druckfest beispielsweise auch bis zu 1 Bar und verformt dann ohne weiteres das Weichgewebe, an dem er anliegt.

Das Erfassungsspektrum der Scanköpfe kann gleich oder unterschiedlich sein.

Jede geeignete elektromagnetische Strahlung ist hier möglich, beispielsweise UV-Licht, sichtbares Licht, Infrarotlicht, Röntgenstrahlung. Es ist aber auch die Erfassung per Ultraschall möglich und es kann bei Bedarf auch ein Scankopf unter Verwendung des Doppler-Effekts realisiert werden.

Die angegebenen technischen Maßnahmen für die Realisierung von Scanköpfen sind lediglich beispielhaft zu verstehen, und jedenfalls nicht einschränkend.

Es ist auch günstig, dass der zweite Scankopf extraoral das Bild des Patienten von vorne erfasst, wenn der Ballon intraoral unter Überdruck gesetzt wird. Hierbei wird unabhängig von Kaubewegungen vom Scan-Kkopf erkannt, an welcher Stelle Weichgewebe vorliegt, und an welcher Stelle die Verformung durch Hartgewebe verhindert oder eingeschränkt wird.

Weitere Einzelheiten, Vorteile und Merkmale ergeben sich aus der nachfolgenden Beschreibung anhand der Zeichnung.

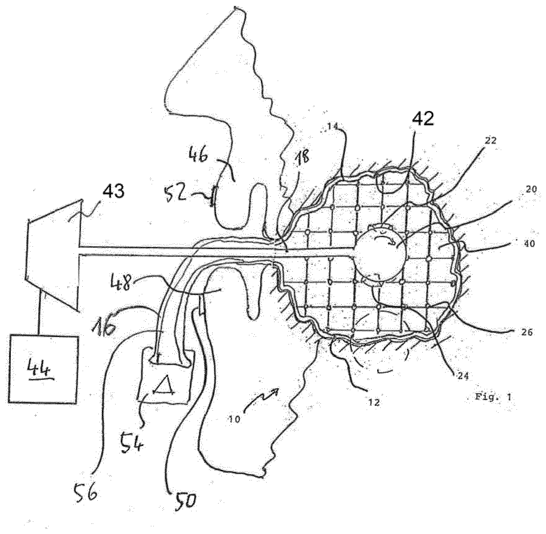

Die einzige Figur der Zeichnung - Fig. 1 - zeigt eine erfindungsgemäße Aufnahmevorrichtung im Einsatz, in einer ersten Ausführungsform.

Aus Fig. 1 ist eine erfindungsgemäße Scanvorrichtung 10 in schematischer Darstellung ersichtlich. Sie weist einen Ballon 12 auf, der aus einer Ballonfolie 14 besteht, die das elastisch dehnbare Material bildet. Der Ballon 12 hat in an sich bekannter Weise einen Ballonanschluss 16. Durch diesen hindurch erstreckt sich ein Scankopfträger 18, die einen Scankopf 20 im Inneren des Ballons hält und sicher führt.

An dem Scankopf 20 sind mehrere Scanner angebracht, von denen hier zwei Scanner 22 und 24 dargestellt sind. Tatsächlich kann auch eine Vielzahl von Scannern vorgesehen sein, beispielsweise 100, während bei einer geringen Anzahl von Scannern diese bevorzugt an dem Scankopf 20 beweglich gelagert sind.

Die Scanner haben die Aufgabe, den gesamten Innenraum der Ballonfolie 14 zu erfassen. Die Ballonfolie 14 weist ein Referenzmuster 26 auf, das im dargestellten Ausführungsbeispiel nach der Art eines Netzes ausgebildet ist, das sich im gleichmäßigen Linienabstand, oder besser noch in einem ungleichmäßigen Muster über die Ballonfolie erstreckt. Insbesondere bei gleichmäßigem Linienabstand ist es auch möglich, die Linien zu codieren, z.B. zu strichlieren, also mit einer je eindeutigen Strichpunktabfolge zu versehen, so dass jede Linien auch bei Lageänderung von Scankopf identifizierbar ist.

Den Scankopfträger 18 umgebend ist eine Dichtung vorgesehen, die den Ballonanschluss 16 gegenüber der Umgebungsluft abdichtet.

Eine Steuervorrichtung 44 ist außerhalb des Ballons vorgesehen. Diese wertet jedenfalls die von den Scankörpern erfassten Bilder aus und steuert in dem dargestellten Ausführungsbeispiel auch einen Überdruck P, mit dem der Ballon 12 aufgeblasen wird.

Durch das Aufblasen legt sich die Ballonfolie 14 eng und genau der Kontur folgend an einen Hohlraum 40 an. Das Referenzmuster 26 erstreckt sich so entlang eines Scanbereichs 42 in verformtem Zustand. Aus der Verformung des Referenzmusters 26 lässt sich die Form des Scanbereichs im Einzelnen berechnen.

In vorteilhafter Ausgestaltung wird der Ballon 12 so aufgeblasen, dass er an dem Hohlraum innen vollständig anliegt. Durch Volumenverkleinerung des Hohlraums erfolgt dann eine weitere Druckzunahme, die über den Scankopf 20 erfassbar ist.

Der Scankopf 20 kann in einem beliebigen geeigneten Wellenlängenbereich betrieben werden. Zu denken ist hier an elektromagnetische Strahlung wie Lichtstrahlung, wozu eine zusätzliche Lichtquelle bevorzugt ist, die den Balloninnenraum ausleuchtet und an dem Scankopf 20 angebracht ist. Auch die Verwendung von Röntgenstrahlung oder von Ultraschall ist anstelle dessen oder zusätzlich möglich.

In einer weiteren Ausführungsform ist es vorgesehen, zunächst ein für die Scanstrahlung transparentes Scanhilfsmittel in den Hohlraum einzuführen und dann aushärten zu lassen.

Das Scanhilfsmittel weist eine Einführöffnung für einen Scankopf auf. Im Hohlraum 40 wird es ausgehärtet, wobei sich das auf der Oberfläche des Scanhilfsmittels aufgebrachte Referenzmuster verformt. Nach einem mindestens teilweisen Aushärten wird es - gegebenenfalls unter elastischem Zusammendrücken oder unter Öffnen des Hohlraums - entnommen, und hieran anschließend wird der Scankopf in das Scanhilfsmittel eingeführt und die Oberfläche des Scanhilfsmittels von innen gescannt.

Erfindungsgemäß ist es vorgesehen, zusätzlich zu dem ersten Scan-Kopf 20 einen zweiten Scankopf 43 extraoral zu realisieren. In dem dargestellten Ausführungsbeispiel ist der zweite Scankopf 43 auf dem gleichen Scankopfträger 18 angebracht. Beide Scanköpfe sind gemeinsam extern gelagert bzw. am Mund des Patienten abgestützt.

Es versteht sich, dass anstelle dessen auch ein stationärer Halter vorgesehen sein kann, der den ersten und/oder den zweiten Scankopf 43 trägt und stützt, so dass der Patient sich aktiv dieser Anordnung nähern muss, um das Scannen zu ermöglichen.

Beide Scanköpfe sind elektrisch und auch von der Steuerung her an einer Steuervorrichtung 44 angeschlossen, die sowohl die Scanvorgänge entweder automatisch oder nach Eingriff durch den Bediener startet und ablaufen lässt aber auch die erfassten Bilder auswertet und die gewonnen Ergebnisse patientenspezifisch zusammenführt.

Der Scankopf 43 ist hierbei auf den Scankopf 18 zu ausgerichtet und erfasst das Bild des geschlossenen/offenen/halboffenen Mundes des Patienten, insbesondere einschließlich der Lippen 46 und 48, bevorzugt aber auch darüber hinaus, also beispielsweise bis zum Bereich der Ohren des Patienten hin.

Der Erfassungswinkel kann in weiten Bereichen an die Erfordernisse angepasst werden. Es ist auch möglich, eine Fokussierungseinrichtung dem zweiten Scankopf vorzuschalten, der nach der Art eines variablen Weitwinkel-Objektivs den erforderlichen Bildausschnitt bereitstellt.

Bevorzugt sind am Tragus, auf den Lippen und/oder aber bei halbgeöffneten Mund im Alveolar-Bereich Markierungselemente 50 und 52 angebracht, deren Lage von dem zweiten Scankopf 43 erfassbar ist.

Der zweite Scankopf 43 erfasst jedenfalls auch Funktionsbewegungen, die der Patient durchführt und ermöglicht so einen Funktionsscan zur Bereitstellung auch dynamischer Daten für die herzustellende Prothese/-n.

Zur Bereitstellung des erwünschten Überdrucks ist eine Druckquelle 54 mit einem Druckschlauch 56 vorgesehen, die an dem Ballon 12 angeschlossen ist und diesen unter Druck setzt, so dass er in der Mundhöhle an dem je dort befindlichen Gewebe, dem sogenannten Scanbereich anliegt.

Es werden nun verschiedene Druckstufen des Ballons von den Scanköpfen 20 und 43 erfasst. Alternativ kann auch der Patient veranlasst werden, den Ballon 12 durch Schließen des Mundes zu komprimieren.

Auch dies führt zur Verformung des je anliegenden Weichgewebes und damit zur Möglichkeit, die Gewebeverteilung zu analysieren und festzustellen.

Der Gegenstand der Erfindung, für den Schutz begehrt wird, ist in den anhängenden Ansprüchen angegeben.

## Patentansprüche

1. Aufnahmevorrichtung, mit einem ersten Scankopf (20), der auf einem Scankopfträger (18) gelagert ist, wobei der Scankopf (20) für das Scannen eines intraoralen Scanbereichs (42), der sich mindestens teilweise um den Scankopf (20) herum erstreckt, ausgebildet ist, wobei ein Ballon (12) aus folienartigem Material (14) vorgesehen ist, welcher unter Überdruck und/oder Adhäsion an dem Scanbereich (42) anliegt, wobei der Ballon (12) mindestens teilweise verformbar ist,
wobei der erste Scankopf (20) im Inneren des Ballons (12) von dem Scankopfträger (18) gehalten ist und ein weiterer extraoraler Scankopf (43) außerhalb des Ballons (12) angeordnet und von dem ersten Scankopf (20) beabstandet ist, wobei die Aufnahmevorrichtung eingerichtet ist, während der Druckbeaufschlagung des folienartigen Materials (14), Verformungen, die in einer Substanz, insbesondere in einem Gewebe, welche Substanz sich im Wesentlichen zwischen dem ersten Scankopf (20) und dem weiteren Scankopf (43) erstreckt, stattfinden, zu scannen.

2. Aufnahmevorrichtung nach Anspruch 1, **dadurch gekennzeichnet, dass** die Substanz als Gewebe, insbesondere Lippengewebe und/oder Wangengewebe, ausgebildet ist und dass mit dem ersten und/oder weiteren Scankopf (43) die Formänderung und/oder die Nachgiebigkeit der Substanz erfasst werden kann und/oder das folienartige Material (14) Muster aufweist, das von dem ersten Scankopf (20) scanbar ist.

3. Aufnahmevorrichtung nach einem der vorhergehenden Ansprüche, **dadurch gekennzeichnet, dass** der Scankopfführer mindestens zwei, insbesondere zwei Scankopfaufnahmen aufweist, und dass eine Scankopfaufnahme stets mit einem Scankopf (20) bestückt ist und die andere Scankopfaufnahme wahlweise mit einem Scankopf bestückbar ist.

4. Aufnahmevorrichtung nach einem der vorhergehenden Ansprüche, **dadurch gekennzeichnet, dass** der erste Scankopf (20) im Wesentlichen als Rundumscankopf ausgebildet ist, mit einem im Wesentlichen kugelförmigen Erfassungsraum und dass der weitere Scankopf (43) als Richtscannkopf ausgebildet ist, der deutlich weniger als eine Halbkugel, insbesondere ein Kegel von etwa 120 Grad Aufweitung erfasst.

5. Aufnahmevorrichtung nach einem der vorhergehenden Ansprüche, **dadurch gekennzeichnet, dass** der weitere Scankopf (43) mit seiner Scanachse im Wesentlichen zum ersten Scankopf (20) hin ausgerichtet ist, insbesondere mit einer Abweichung von weniger als 30 Grad.

6. Aufnahmevorrichtung nach einem der vorhergehenden Ansprüche, **dadurch gekennzeichnet, dass** die Scanköpfe auf einem gemeinsamen Scankopfträger (18) in voneinander beabstandeten und genau definierten Position je in einer Scankopfaufnahme aufgenommen sind.

7. Aufnahmevorrichtung nach einem der vorhergehenden Ansprüche, **dadurch gekennzeichnet, dass** das folienartige Material (14), das den ersten Scankopf (20) mindestens teilweise umgibt, austauschbar angebracht ist und dass der erste Scankopf (20) durch eine Öffnung in dem folienartigen Material (14) hindurchführbar ist.

8. Aufnahmevorrichtung nach einem der vorhergehenden Ansprüche, **dadurch gekennzeichnet, dass** das folienartige Material (14) nach Verbringen auf einem vorgegebenen Überdruck durch Verkleinerung eines Hohlraums (40), der den Scanbereich (42) umgibt, komprimierbar ist und dass die Bewegung des Scanbereichs (42) als Funktion des bestehenden Drucks von dem ersten Scankopf (20) erfassbar ist.

9. Aufnahmevorrichtung nach einem der vorhergehenden Ansprüche, **dadurch gekennzeichnet, dass** die Aufnahmevorrichtung eine Steuer- und Auswertevorrichtung aufweist, die geeignet ist, Scanergebnisse basierend auf dem je eingeleiteten Überdruck bzw. Druck unter dem das folienartige Material (14) steht, zu erfassen und auszuwerten.

10. Aufnahmevorrichtung nach einem der vorhergehenden Ansprüche, **dadurch gekennzeichnet, dass** mindestens zwei Aufblaszustände oder Druckzustände des folienartigen Materials (14) je einem Scanvorgang zu Grunde gelegt werden und dass eine Steuer- und Auswertevorrichtung der Aufnahmevorrichtung geeignet ist, einzelne Teile des Scanbereichs (42) zwischen den beiden zu erfassen und/oder Unterschiede zwischen den beiden Zuständen zu erfassen.

11. Aufnahmevorrichtung nach einem der vorhergehenden Ansprüche, **dadurch gekennzeichnet, dass** basierend auf mehreren Scanvorgängen des ersten und/oder des weiteren Scankopfs (43) ein digitales Modell eines Patientenkopfes hinsichtlich der anatomischen Gegebenheiten und der Nachgiebigkeit des je betroffenen Gewebes, das den Scanbereich (42) bildet, erstellt werden kann.

12. Aufnahmevorrichtung nach einem der vorhergehenden Ansprüche, **dadurch gekennzeichnet, dass** jeder Scankopf (20) eine Erfassungsmöglichkeit für UV-Licht, für sichtbares Licht und/oder für Infrarotlicht und/oder für Ultraschall und/oder für Röntgenstrahlen bietet, insbesondere je nach unterschiedlichen Spektren getrennt.

13. Aufnahmevorrichtung nach einem der vorhergehenden Ansprüche, **dadurch gekennzeichnet, dass** eine Steuer- und Auswertevorrichtung in der Aufnahmevorrichtung vorgesehen ist, mit welcher von einem extraoralen Scankopf (43) eine Bipupilar-Linie inklusive mindestens eines Referenzmarkers erfassbar ist.

14. Aufnahmevorrichtung nach einem der vorhergehenden Ansprüche, **dadurch gekennzeichnet, dass** die Aufnahmevorrichtung eine Steuer-/ und Auswertvorrichtung aufweist, mit welcher die anatomischen Gegebenheiten wie ein (zahnloser) Kieferkamm und/oder eine Verformung der Lippen und des umgebenden Bereichs eines Patienten erfassbar ist, wenn das folienartige Material (14) nach der Art des Ballon (12) intraoral unter Überdruck gesetzt wird.

15. Verfahren für den Betrieb einer Aufnahmevorrichtung, die einen ersten Scankopf (20) aufweist, der auf einem Scankopfträger (18) gelagert ist, wobei der Scankopf (20) einen Scanbereich (42), der sich mindestens teilweise um den Scankopf (20) herum erstreckt aufweist, wobei der Scanbereich (42) mit einem folienartigen Material (14) in Form eines Ballons (12) versehen ist, das unter Überdruck gesetzt wird und durch den Überdruck und/oder durch Adhäsion an dem Scanbereich (42) anliegt, so dass sich der Ballon (12) mindestens teilweise verformt,
wobei der erste Scankopf (20) im Inneren des Ballons (12) von dem Scankopfträger (18) gehalten wird und ein weiterer extraoraler Scankopf (43) von dem ersten Scankopf (20) außerhalb des Ballons (12) beabstandet angeordnet wird, wobei während der Druckbeaufschlagung des folienartigen Materials (14), Verformungen, die in einer Substanz, insbesondere in einem Gewebe, welche Substanz sich im Wesentlichen zwischen dem ersten und dem weiteren Scankopf (43) erstreckt, stattfinden, gescannt werden.

## Claims

1. A recording device, having a first scan head (20) which is mounted on a scan head carrier (18), the scan head (20) being configured for scanning an intraoral scanning area (42) which extends at least partially around the scan head (20), wherein a balloon (12) made of a film-like material (14) is provided which abuts against the scanning area (42) under overpressure and/or adhesion, wherein the balloon (12) is at least partially deformable, wherein the first scan head (20) is held inside the balloon (12) by the scan head carrier (18) and a further extraoral scan head (43) is arranged outside the balloon (12) and spaced apart from the first scan head (20), wherein the recoding device is set to scan deformations during pressure application to the film-like material (14), which deformations occur in a substance, in particular a tissue, which substance substantially extends between the first scan head (20) and the further scan head (43).

2. The recording device according to claim 1, **characterized in that** the substance is configured as a tissue, in particular a lip tissue and/or cheek tissue, and **in that** the first and/or further scan head (43) can detect the change in shape and/or the resilience of the substance, and/or the film-like material (14) comprises patterns (14) which can be scanned by the first scan head (20) .

3. The recording device according to one of the preceding claims, **characterized in that** the scan head guide has at least two, in particular two, scan head accommodations, and **in that** one scan head accommodation is always equipped with a scan head (20) and the other scan head accommodation can optionally be equipped with a scan head.

4. The recording device according to one of the preceding claims, **characterized in that** the first scan head (20) is substantially configured as an all-round scan head having a substantially spherical range of detection and **in that** the further scan head (43) is configured as a directional scan head detecting significantly less than one hemisphere, in particular a cone of approximately 120 degrees expansion.

5. The recording device according to one of the preceding claims, **characterized in that** the further scan head (43), with its scanning axis, is substantially aligned towards the first scan head (20), in particular with a deviation of less than 30 degrees.

6. The recording device according to one of the preceding claims, **characterized in that** the scan heads are each accommodated on a common scan head carrier (18) in a spaced apart and precisely defined position in a scan head accommodation.

7. The recording device according to one of the preceding claims, **characterized in that** the film-like material (14) which at least partially surrounds the first scan head (20) is replaceably mounted and that the first scan head (20) can be passed through an opening in the film-like material (14).

8. The recording device according to one of the preceding claims, **characterized in that** the film-like material (14) is compressible after being brought to a predetermined overpressure by decreasing a cavity (40) surrounding the scanning area (42) and that the first scan head (20) can detect the movement of the scanning area (42) as a function of the existing pressure.

9. The recording device according to one of the preceding claims, **characterized in that** the recording device comprises a control and evaluation device which is suitable for detecting and evaluating scan results based on the respective overpressure or pressure applied to the film-like material (14).

10. The recording device according to one of the preceding claims, **characterized in that** at least two inflation modes or pressure modes of the film-like material (14) are each used as a basis for a scanning operation, and **in that** a control and evaluation device of the recording device is suitable for detecting individual parts of the scanning area (42) between the two modes and/or for detecting differences between the two modes.

11. The recording device according to one of the preceding claims, **characterized in that** a digital model of a patient's head can be created based on several scanning operations of the first and/or the further scan head (43) with regard to anatomical conditions and resilience of the respective tissue concerned which forms the scanning area (42).

12. The recording device according to one of the preceding claims, **characterized in that** each scan head (20) provides a detection possibility for UV light, visible light and/or infrared light and/or ultrasound and/or X-rays, in particular separated by different spectra.

13. The recording device according to one of the preceding claims, **characterized in that** a control and evaluation device is provided in the recording device by the use of which a bipupillary line including at least one reference marker can be detected by an extraoral scan head (43).

14. The recording device according to one of the preceding claims, **characterized in that** the recording device has a control and evaluation device by the use of which the anatomical conditions such as a (toothless) jaw ridge and/or a deformation of the lips and the surrounding region of a patient can be detected when the film-like material (14) is placed under intraoral overpressure according to the type of balloon (12).

15. A method for operating a recording device comprising a first scan head (20) mounted on a scan head carrier (18), the scan head (20) having a scanning area (42) extending at least partially around the scan head (20), wherein the scanning area (42) is provided with a film-like material (14) in the form of a balloon (12) which is pressurized and abuts against the scanning area (42) due to the overpressure and/or adhesion so that the balloon (12) deforms at least partially, wherein the first scan head (20) is held inside the balloon (12) by the scan head carrier (18) and a further extraoral scan head (43) is arranged spaced apart from the first scan head (20) outside of the balloon (12), wherein, while applying pressure to the film-like material (14), deformations are scanned which occur in a substance, in particular in a tissue, which substance extends substantially between the first and the further scan head (43).

## Revendications

1. Dispositif de réception qui comprend une première tête de balayage (20) qui est montée sur un support de tête de balayage (18), où la tête de balayage (20) est conçue pour le balayage d'une zone de balayage intraorale (42) qui s'étend au moins partiellement autour de la tête de balayage (20), où un ballon (12) en matériau de type film (14) qui s'applique sous surpression et/ou par adhérence contre la zone de balayage (42) est prévu, où le ballon (12) est au moins partiellement déformable, où la première tête de balayage (20) est maintenue à l'intérieur du ballon (12) par le support de tête de balayage (18) et une autre tête de balayage extraorale (43) est disposée à l'extérieur du ballon (12) et à distance de la première tête de balayage (20), où le dispositif de réception est adapté pour balayer, pendant la mise sous pression du matériau de type feuille (14), des déformations qui ont lieu dans une substance, en particulier dans un tissu, cette substance s'étendant essentiellement entre la première tête de balayage (20) et l'autre tête de balayage (43).

2. Dispositif de réception selon la revendication 1, **caractérisé en ce que** la substance est conçue comme un tissu, en particulier un tissu de la lèvre et/ou un tissu de la joue, et **en ce que** la première et/ou l'autre tête de balayage (43) permet de détecter la modification de forme et/ou la souplesse de la substance et/ou le matériau en forme de feuille (14) présente des motifs qui peuvent être balayés par la première tête de balayage (20) .

3. Dispositif de réception selon l'une des revendications précédentes, **caractérisé en ce que** le guide de tête de balayage présente au moins deux, en particulier deux logements de tête de balayage, et **en ce qu'**un logement de tête de balayage est toujours équipé d'une tête de balayage (20) et l'autre logement de tête de balayage peut être équipé au choix d'une tête de balayage.

4. Dispositif de réception selon l'une des revendications précédentes, **caractérisé en ce que** la première tête de balayage (20) est conçue essentiellement comme une tête de balayage circulaire, avec un espace de détection essentiellement sphérique, et **en ce que** l'autre tête de balayage (43) est conçue comme une tête de balayage directionnelle, qui détecte nettement moins qu'une demisphère, en particulier un cône d'environ 120 degrés d'élargissement.

5. Dispositif de réception selon l'une des revendications précédentes, **caractérisé en ce que** l'axe de balayage de l'autre tête de balayage (43) est orienté essentiellement vers la première tête de balayage (20), notamment avec un écart inférieur à 30 degrés.

6. Dispositif de réception selon l'une des revendications précédentes, **caractérisé en ce que** les têtes de balayage sont reçues sur un support de tête de balayage commun (18) dans des positions espacées les unes des autres et définies avec précision, chacune dans un logement de tête de balayage.

7. Dispositif de réception selon l'une des revendications précédentes, **caractérisé en ce que** le matériau en forme de feuille (14) entourant au moins partiellement la première tête de balayage (20) est monté de manière interchangeable et **en ce que** la première tête de balayage (20) peut être passée à travers une ouverture dans le matériau en forme de feuille (14).

8. Dispositif de réception selon l'une des revendications précédentes, **caractérisé en ce que** le matériau en forme de feuille (14), après avoir été amené à une surpression prédéterminée, peut être comprimé par réduction d'une cavité (40) qui entoure la zone de balayage (42) et **en ce que** le mouvement de la zone de balayage (42) peut être détecté par la première tête de balayage (20) en fonction de la pression existante.

9. Dispositif de réception selon l'une des revendications précédentes, **caractérisé en ce que** le dispositif de réception présente un dispositif de commande et d'évaluation qui est apte à détecter et à évaluer les résultats de scannage sur la base de la surpression ou de la pression introduite à chaque fois dans le matériau en forme de feuille (14).

10. Dispositif de réception selon l'une des revendications précédentes, **caractérisé en ce qu'**au moins deux états de gonflage ou deux états de pression du matériau en feuille (14) sont respectivement à la base d'une opération de balayage et **en ce qu'**un dispositif de commande et d'évaluation du dispositif de réception est approprié pour détecter des parties individuelles de la zone de balayage (42) entre les deux et/ou pour détecter des différences entre les deux états.

11. Dispositif de réception selon l'une des revendications précédentes, **caractérisé en ce que**, sur la base de plusieurs processus de balayage de la première et/ou de l'autre tête de balayage (43), un modèle numérique de la tête d'un patient peut être créé en ce qui concerne les données anatomiques et la souplesse du tissu concerné qui forme la zone de balayage (42).

12. Dispositif de réception selon l'une quelconque des revendications précédentes, **caractérisé en ce que** chaque tête de balayage (20) offre une possibilité de détection de la lumière UV, de la lumière visible et/ou de la lumière infrarouge et/ou des ultrasons et/ou des rayons X, notamment séparément selon des spectres différents.

13. Dispositif de réception selon l'une des revendications précédentes, **caractérisé en ce qu'**un dispositif de commande et d'évaluation est prévu dans le dispositif de réception, avec lequel une ligne bipupillaire, y compris au moins un marqueur de référence, peut être détectée par une tête de balayage extra-orale (43).

14. Dispositif de réception selon l'une des revendications précédentes, **caractérisé en ce que** le dispositif de réception présente un dispositif de commande et d'évaluation avec lequel les données anatomiques telles qu'une crête (édentée) et/ou une déformation des lèvres et de la zone environnante d'un patient peut être détectées lorsque le matériau en forme de feuille (14) est mis en surpression intrabuccale à la manière d'un ballon (12).

15. Procédé de fonctionnement d'un dispositif de réception qui comprend une première tête de balayage (20) montée sur un support de tête de balayage (18), où la tête de balayage (20) comprend une zone de balayage (42) qui s'étend au moins partiellement autour de la tête de balayage (20), où la zone de balayage (42) est pourvue d'un matériau de type film (14) sous la forme d'un ballon (12) qui est mis en surpression et qui s'applique contre la zone de balayage (42) par la surpression et/ou par adhérence, de manière à déformer au moins partiellement le ballon (12), où la première tête de balayage (20) est maintenue à l'intérieur du ballon (12) par le support de tête de balayage (18) et une autre tête de balayage extra-buccale (43) est disposée à distance de la première tête de balayage (20) à l'extérieur du ballon (12), où pendant la mise sous pression du matériau en forme de feuille (14), des déformations qui ont lieu dans une substance, en particulier dans un tissu, cette substance s'étendant essentiellement entre la première et l'autre tête de balayage (43), sont scannées.
